# EUROPEAN PATENT APPLICATION

(11) **EP 1 974 733 A1**
(43) Date of publication of application: **01.10.2008**
(21) Application number: 07104792.2
(22) Date of filing: 23.03.2007
(51) Int. Cl.: A61K 33/34, A61K 9/50, A61K 9/127, A61P 27/02

(54) **Use of a masked or coated copper salt for the treatment of macular degeneration**

(71) Applicant: Novartis AG, 4056 Basel (CH)
(72) Inventor: The designation of the inventor has not yet been filed
(74) Representative: von Sprecher, Georg

(57) **Abstract**

The present invention relates to a method of protecting copper-sensitive compounds and/or compositions from decomposition, by using a masked or coated copper salt.
Preferably, the copper is coated with gelatin, a liposome, or a mono- and di- glyceride.

## Description

The present invention relates to a method of protecting copper-sensitive compounds and/or compositions from decomposition.

Omega-3-fatty acids and a number of vitamins and other pharmaceutically effective compounds or compositions are susceptible to decomposition when in contact with copper salts. This decomposition is primarily a problem during storage of such compounds and / or compositions and in particular when water is present. Said presence of water applies also to minute amounts of water, such as humidity per se, traces of water in one of the components of a composition and/or of a compound, or from environmental humidity.

There are a number of compounds and/or compositions, which contain a copper salt as an important and hence often mandatory ingredient. In such a situation, the interaction of a copper salt and the copper sensitive compound and/or composition is controlled by the provision(s) provided in the next paragraphs, i.e. decomposition of the copper sensitive compound and/or composition is substantially inhibited or typically prevented.

In a first aspect, the problem is solved by providing the copper salt with a proper masking or coating. This masking or coating typically suppresses an interaction with said copper sensitive compound and/or composition. The copper salt is for example masked or coated with - or embedded in - gelatin or liposomes and/or both such as for example a coated copper salt is embedded in gelatin. There are other ways of protecting copper salts, e.g. by encapsulating it with mono- and di-glycerides, as for example known from the product Descote® which is commercially available.
Copper salts can form a stable complex with an appropriate cyclodextrin compound, which would typically release its free copper salt at a later stage, e.g. when deemed required e.g. upon enzymatic resolution of the complex in the gut.
Copper salts might be coated with a zwitterionic phospholipid including but not limited to phosphatidylcholine, phosphatidylserine, phosphatidylethanolamine, spingomyelin and other ceramides, as well as various other zwitterionic phospholipids.

In the enclosed examples there are a number of copper sensitive compounds which decompose if the copper is not properly masked or coated. Such a copper sensitive composition may for example contain:

### Example 1

| | |
|---|---|
| Vitamin C 500mg | Calcium ascorbate dihydrate, USP |
| Vitamin E 400 (mg) | Alpha tocopheryl acetate, USP |
| Zinc 40mg | Zinc oxide |
| Copper 1 mg | Cupric carbonate, basic, in acc. to Merck Index |
| Omega 3 fatty acid | Total 350mg (DHA : EPA ratio from 1 : 4, 1 : 3, |
| 1 : 2, 1:1, 2 : 1, 3 : 1, or4 : 1, | |
| Omega-3 acid triglycerides Ph.Eur. 120mg | |
| Lutein 10 mg | FloraGlo 20% natural source |
| Zeaxanthin 2mg | from Lutein |

### Example 2

In another experiment, the cupric carbonate, basic, is incorporated in the shell of a capsule which contains the below ingredients. Specifically, the excipients for making such a shell are for example:

| | |
|---|---|
| Gelatin | 175 bloom bovine |
| Glycerin | 99%, USP |
| Soybean oil flakes | (hydrogenated), NF |
| Soybean oil | USP, and |
| Colorants (if required), | organic and/or inorganic |

The stability of the above composition is investigated in a setup where the copper is not masked, and in a situation wherein the copper is masked in the shell, and hence not in direct contact with the copper sensitive ingredients. The stability of the described masking /coating method correlates with the degree of decomposition of such a copper sensitive compound and/or composition.

### Example 3

| | |
|---|---|
| 240 mg Fish oil with 70% DHA | label 160 mg Omega 3 fatty acids |
| Docosahexaeonic acid (DHA) | label 130 mg DHA |
| Eicosapentaenoic acid (EPA) as part of fish oil | label 11 mg |
| 91.5mg Calcium ascorbate | label 60 mg Vitamin C |
| 31.343mg d,l Alpha-tocopheryl acetate | label 20 mg Vitamin E |
| 55mg Lutein 20% in safflower oil | label 10 mg Lutein |
| 12.447mg Zinc oxide | label 10 mg Zinc |
| 0.45mg Cupric carbonate basic | label 0.25 mg Copper |
| Zeaxanthin as part of Lutein | label 0.8 mg |

The above composition may contain other excipients for the production of a targeted galenic formulation, e.g. for making a shell of a capsule or the like.

### Bibliographic Convention:

As used herein, copper or a copper salt are used interchangeably and pertain preferably Cu²⁺ but may also include Cu⁺ . A copper salt comprises preferably a pharmaceutically acceptable anion such as chloride, oxide, hydroxide, gluconate, carbonate, or the like.

As used herein, an omega-3-fatty acid are mainly but not only eicosapentaenoic acid (EPA) and docosahexaenoic acid (DHA).

As used herein, the term masked, coated or embedded copper or copper salt pertains to a copper salt and a masking, coating or embedding agent, which effectively prevents a direct interaction of said copper salt with said copper sensitive compound and/or composition.

As used herein, a digestible capsule material is for example the shell material described in the above Example 2, but is in general any capsule or capsule shell or shell material being used in the state-of-the-art for pharmaceutical / dietary / nutraceutical capsules, typically being organic polymeric compositions and typically being pharmaceutically compatible (nontoxic, bio-degradable, digestible in the gut or stomach as deemed suitable).

In another aspect the present invention relates to the use of a masked or coated copper salt in a pharmaceutical composition for the treatment of a disease being treatable by a copper salt, e.g. age related macular degeneration (AMD).

The invention further relates to the use of a masked or coated copper salt in a method to stabilize a pharmaceutical composition.

The invention further relates to a storage stable pharmaceutical composition comprising an omega-3-fatty acid and a coated or masked copper salt.

The invention further relates to use a masked or coated copper salt in a method to stabilize a food supplement and/or nutraceutical composition from copper salt dependent decomposition.

The invention further relates to the use of a masked or coated copper salt in a method to stabilize a pharmaceutical composition from copper salt dependent decomposition.

## Claims

1. The use of a masked or coated copper salt in a pharmaceutical composition for the treatment and/or prevention of a disease being treatable and/or preventable by a copper salt.

2. The use of claim 1, wherein said disease is age related macular degeneration (AMD).

3. The use of a masked or coated copper salt in a method to stabilize a pharmaceutical composition from copper salt dependent decomposition.

4. The use in accordance to any of the preceding claims wherein said pharmaceutical composition further comprises an omega-3-fatty acid.

5. The use in accordance to any of the preceding claims wherein said copper is coated with gelatin, a liposome, or a mono- and di-glyceride.

6. The use in accordance to any of the preceding claims wherein said copper is copper gluconate, copper oxide, copper carbonate or copper chloride.

7. The use in accordance to any of the preceding claims wherein said coating is a zwitterionic phospholipid including but not limited to phosphatidylcholine, phosphatidylserine, phosphatidylethanolamine, spingomyelin and other ceramides, as well as various other zwitterionic phospholipids.

8. The use in accordance to any of the preceding claims wherein said copper is a copper cyclodextrin complex.

9. Digestible capsule material, in particular a gastrointestinal tract digestible capsule material, containing a masked and/or coated copper salt, **characterized in that** said capsule material represent said masking and/or coating material for said copper salt.

10. Digestible capsule material of claim 9, wherein said copper salt is already coated with a coating, and said coating might be the same or another masking material than said capsule material.

11. The use of a masked or coated copper salt, said masking being obtained in accordance to the above description, in a nutrient, or in a dietary supplement, in particular in a purpose as described hereinbefore, and optionally in conjunction with an AMD treatment, such as treatment with Lucentis® or Visudyne®.

12. The use of a masked or coated copper salt in accordance to the above description as a nutrient composition, or as a dietary supplement composition, in the prevention/treatment of AMD.
